# EUROPÄISCHE PATENTSCHRIFT

(11) **EP 0 705 839 B1**
(45) Veröffentlichungstag und Bekanntmachung des Hinweises auf die Patenterteilung: **05.12.2001**
(21) Anmeldenummer: 95115276.8
(22) Anmeldetag: 28.09.1995
(51) Int. Cl.: C07F 9/24, A61K 31/66, C07F 9/22, C07F 9/58, C07F 9/6533

(54) **Imidobisphosphorsäuren, Verfahren zu ihrer Herstellung und Verwendung derselben**
Imidobisphosphoric acids, process for their preparation and their use
Acides imido bis phosphoriques, leur procédé de préparation et leur utilisation

(30) Priorität: 04.10.1994 DE 4435496
(43) Veröffentlichungstag der Anmeldung: 10.04.1996
(73) Patentinhaber: HOECHST AKTIENGESELLSCHAFT, 65929 Frankfurt am Main (DE)
(72) Erfinder: Naumann, Christoph, Dr., D-65527 Niederhausen (DE); Yoshikawa, Katsuhiro, Saitama 350-11 (JP); Kitamura, Kazuyuki, Iruma-shi, Saitama 358 (JP); Inazu, Mizuho, Iruma-shi, Saitama 358 (JP)

(56) Entgegenhaltungen:
- EP-A- 0 620 227
- WO-A-92/11269
- DE-B- 1 041 044
- US-A- 2 798 086
- CHEMICAL ABSTRACTS, vol. 108, no. 19, 9.Mai 1988 Columbus, Ohio, US; abstract no. 167599, DING Y ET AL 'Organophosphorus compounds. XXI. Regioselective phosphorylation of ambident anions' & HUAXUE XUEBAO (HHHPA4,05677351);87; VOL.45 (8); PP.783-90, ACAD. SIN.;SHANGHIA INST. ORG. CHEM.; SHANGHAI; PEOP. REP. CHINA (CN),
- ZH. OBSHCH. KHIM. (ZOKHA4);75; VOL.45 (11); PP.2394-6, INST. ORG. KHIM.;KIEV; USSR, PINCHUK A M ET AL 'Reaction of N,N-dichloroamines with phosphites'
- PHOSPHORUS, SULFUR SILICON RELAT. ELEM. (PSSLEC,10426507);94; VOL.91 (1-4); PP.169-77, HOECHST AG;FRANKFURT; D-65926; GERMANY (DE), NAUMANN C ET AL 'Synthesis of new imidobisphosphoric acids'

## Beschreibung

Osteoporose ist eine häufig vorkommende Knochenerkrankung. Bei den verschiedenen Formen der Osteoporose kommt es zu einem starken Verlust an Knochengewebe, so daß schließlich die mechanische Stabilität des Knochens verloren geht. In gesunden Menschen ist die Rate mit der Osteoclasten und Osteoplasten gebildet werden so ausgebildet, daß Knochenbildung und Knochenresorption im Gleichgewicht stehen. Bei Osteoporose ist das Gleichgewicht gestört, so daß es zu einem Knochenabbau kommt.

In EP-A 0 354 806, EP-A 0 546 548, WO-A-921 und EP-A 0 522 576 wird die Verwendung von Bisphosphonsäuren zur Behandlung von Knochenerkrankungen wie beispielsweise Osteoporose beschrieben.

Ding Yixiang et al., Huaxue Xuebao, 45(8), 783-90 (1987); Riesel et al., Z. Anorg. Allg. Chem. 430, 227-233 (1977); Pinchuk et al., J. Gen. Chem. USSR, 45, 1975, 2352-2354; Arbuzov et al., IZU Akad. Nauk. SSSR Ser. Khim., 1956, 953-957 und DBP 10 41 044 beschreiben Synthesen zur Herstellung von Imidobisphosphorsäurealkylester und aus US-Patent Nr. 2,798,086 sowie Arbuzov et al., IZU Akad. Nauk. SSSR Ser. Khim., 1954, 913-916 ist die Verwendung einzelner Imidobisphosphorsäurealkylester als Insektizid bekannt.

In dem Bestreben wirksame Verbindungen zur gleichzeitigen Behandlung und Prophylaxe von degenerativen Gelenkserkrankungen und Osteoporose mit geringen Nebenwirkungen zu erhalten, wurde nun überraschenderweise gefunden, daß die erfindungsgemäßen Imidobisphosphorsäuren in vitro die Calcium-Freisetzung aus Hydroxylapatit hemmen.

Die Erfindung betrifft daher Verbindungen der Formel I, 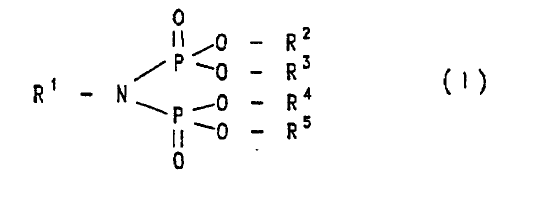 sowie deren physiologisch verträglichen Salze, wobei
- R¹: (C₁-C₁₀)-Alkyl, (C₁-C₈)-Alkanoyl, (C₆-C₁₀)-Aryl oder Het bedeutet, die ein- bis sechsfach substituiert sein können durch
Halogen, -OH, -NH₂, -NH-CO-R⁷, -O-CO-R⁷ und -N-(R⁸)₂,
wobei R⁷ (C₁-C₆)-Alkyl und R⁸ unabhängig voneinander Wasserstoff oder (C₁-C₃)-Alkyl ist, oder
für den Rest der Formel II steht 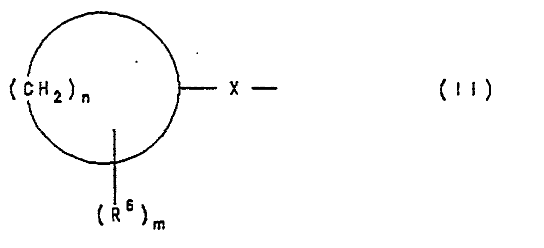 worin
n eine ganze Zahl von 3 bis 10,
m Null bis 3,
R⁶ unabhängig voneinander (C₁-C₆)-Alkyl, das gegebenenfalls ein- bis sechsfach substituiert ist durch Halogen oder -NH-CO-R⁷ mit R⁷ = (C₁-C₆)-Alkyl bedeuten und
X abwesend ist oder (C₁-C₁₀)-Alkyl bedeutet, und
R², R³, R⁴ oder R⁵ unabhängig voneinander für Wasserstoff, (C₁-C₅)-Alkyl, Lithium, Natrium, Kalium oder Si(R)₃, mit R = (C₁-C₅)-Alkyl, stehen,
dabei ausgenommen sind Verbindungen der Formel I mit
R¹ = Methyl und R² - R⁵ gleichzeitig = Methyl, Ethyl, Propyl, Isopropyl, Butyl oder Isobutyl oder R² und R³ = Ethyl oder Isopropyl und R⁴ und R⁵ = Methyl,
R¹ = Ethyl und R² - R⁵ gleichzeitig = Ethyl, Propyl, Isopropyl, Butyl oder Isobutyl oder R² und R³ = Ethyl und R⁴ und R⁵ = Methyl,
R¹ = Isopropyl und R² und R³ = Isopropyl und R⁴ und R⁵ = Methyl,
R¹ = Butyl und R² - R⁵ gleichzeitig = Ethyl, Isopropyl oder Butyl,
R¹ = Isobutyl und R² - R⁵ gleichzeitig = Ethyl,
R¹ = Pentyl und R² - R⁵ gleichzeitig = Ethyl, Propyl oder Butyl,
R¹ = 1-tert. Butyl-1,1'-dimethyl-propyl und R² - R⁵ gleichzeitig = Ethyl,
R¹ = Dodecyl und R² - R⁵ gleichzeitig = Butyl,
R¹ = Phenyl und R² - R⁵ gleichzeitig = Propyl, und
R¹ = Cyclohexyl und R² - R⁵ gleichzeitig = Ethyl.

Unter dem Rest der Formel II werden Reste wie beispielsweise Cyclopropyl, Cyclobutyl, Cyclopentyl, Cyclohexyl, Cycloheptyl, Cyclooctyl, Cyclononyl oder Cyclodecyl verstanden, die gegebenenfalls durch eine oder mehrere Alkylreste mit 1 bis 6 Kohlenstoffatomen substituiert sein können.

Unter Het werden monocyclische 5- bis 6-gliedrige oder bicyclische 8- bis 10-gliedrige gesättigte, ungesättigte oder teilweise ungesättigte heterocyclische Reste mit 1 bis 3 gleichen oder verschiedenen Heteroatomen aus der Reihe Stickstoff, Sauerstoff und Schwefel verstanden. Bevorzugt enthält Het 1 bis 2 Stickstoffatome.

Monocyclische heterocyclische Reste sind z.B. Pyrrol, Pyrrolin, Pyrrolidin, Pyridin, Tetrahydropyridin, Piperidin, Pyrazol, Imidazol, Pyrazolin, Imidazolin, Pyrazolidin, Imidazolidin, Pyridazin, Pyrimidin, Pyrazin, Piperazin, Oxazol, Oxazolin, Oxazolidin, Isoxazol, Isoxazolin, Isoxazolidin, Morpholin, Thiazol, Isothiazol, Thiazolin, Isothiazolin, Thiazin, Furan, Tetrahydrofuran, Thiophen, Thiolan, Dioxan, Pyran, Thiopyran, 1.3-Dioxolen, 1.2-Oxathiolan, 1.2.3-Triazol, 1.2.4-Triazol, 1.2.4-Thiadiazol, 1.3.4.-Thiadiazol, 1.3.5.-, 1.2.4- und 1.2.3-Triazin. Bevorzugt sind: Pyrrol, Pyrrolin, Pyrrolidin, Pyridin, Tetrahydropyridin, Piperidin, Pyrazol, Imidazol, Pyrazolin, Imidazolin, Pyrazolidin, Imidazolidin, Pyridazin, Pyrimidin, Pyrazin, Piperazin, Morpholin und 1.3.5-Triazin.

Bicyclische heterocyclische Reste sind z.B. Benzothiophen, Benzofuran, Indol, Isoindol, Indolizin, Indazol, Benzimidazol, Chinolin, Isochinolin, Phthalazin, Chinoxalin, Chinazolin, Cinnolin, Chroman, 4H-Chromen, Cumaron, Thionaphthen, Pyrrolo[1.5-a]pyrimidin, lmidazo[2.1-b]oxazol, Purin und Pteridin.

Alkyl kann geradkettig oder verzweigt sein. Entsprechendes gilt für davon abgeleitete Reste wie z.B. Alkanoyl.

Falls nicht anders angegeben steht Halogen für Chlor, Brom, Jod oder Fluor, bevorzugt für Fluor, Chlor oder Brom.

Bevorzugt sind die Verbindungen der Formel I, wobei
- R¹: (C₁-C₁₀)-Alkyl, (C₁-C₈)-Alkanoyl, (C₆-C₁₀)-Aryl oder Het mit 1 oder 2 Stickstoffatomen bedeutet, die ein- bis sechsfach substituiert sein können durch
Halogen, -OH, -NH₂, -NH-CO-R⁷, -O-CO-R⁷ und -N-(R⁸)₂,
wobei R⁷ (C₁-C₆)-Alkyl und R⁸ unabhängig voneinander Wasserstoff oder (C₁-C₃)-Alkyl ist, oder
für den Rest der Formel II steht 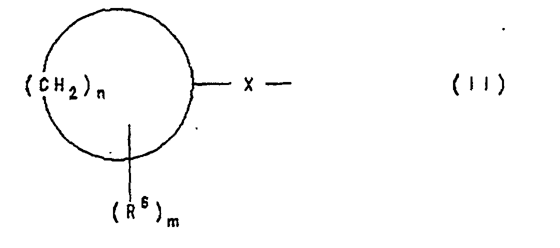 worin
n eine ganze Zahl von 3 bis 10,
m 1,
R⁶ (C₁-C₆)-Alkyl, das gegebenenfalls ein- bis sechsfach substituiert ist durch Halogen oder -NH-CO-R⁷ mit R⁷ = (C₁-C₆)-Alkyl bedeuten und
X abwesend ist oder (C₁-C₁₀)-Alkyl bedeutet, und
R² und R³, R⁴ und R⁵ für Wasserstoff, Lithium, Natrium oder (C₁-C₅)-Alkyl stehen,
dabei ausgenommen sind Verbindungen der Formel I mit
R¹ = Methyl und R² - R⁵ gleichzeitig = Methyl, Ethyl, Propyl, Isopropyl, Butyl oder Isobutyl oder R² und R³ = Ethyl oder Isopropyl und R⁴ und R⁵ = Methyl,
R¹ = Ethyl und R² - R⁵ gleichzeitig = Ethyl, Propyl, Isopropyl, Butyl oder Isobutyl oder R² und R³ = Ethyl und R⁴ und R⁵ = Methyl,
R¹ = Isopropyl und R² und R³ = Isopropyl und R⁴ und R⁵ = Methyl,
R¹ = Butyl und R² - R⁵ gleichzeitig = Ethyl, Isopropyl oder Butyl,
R¹ = Isobutyl und R² - R⁵ gleichzeitig = Ethyl,
R¹ = Pentyl und R² - R⁵ gleichzeitig = Ethyl, Propyl oder Butyl,
R¹ = 1-tert. Butyl-1,1'-dimethyl-propyl und R² - R⁵ gleichzeitig = Ethyl,
R¹ = Phenyl und R² - R⁵ gleichzeitig = Propyl, und
R¹ = Cyclohexyl und R² - R⁵ gleichzeitig = Ethyl.

Besonders bevorzugt sind Verbindungen der Formel I, wobei
- n: für eine ganze Zahl 7, 8, 9 oder 10 steht,
- m: 1 bedeutet,
- R¹: für (C₁-C₅)-Alkyl, Phenyl, Naphthyl oder einen Rest aus der Gruppe Piperidinyl, Pyrolidinyl, Piperazinyl, Pyridinyl, Morpholinyl, Imidazol und Pyrazol steht,
- R², R³, R⁴ und R⁵: gleichzeitig für Wasserstoff, Natrium oder Lithium stehen.

Insbesondere bevorzugt sind die Verbindungen aus folgender Gruppe:
N-Cycloheptyl-imidobisphosphorsäuretetraethylester,
N-[2-(N',N'-diisopropylamino)ethyl]-imidobisphosphorsäuretetraethylester,
N-[2-(N'-Morpholino)-ethyl]-imidobisphosphorsäuretetraethylester,
N-[2-(2-Pyridyl)-ethyl]-imidobisphosphorsäuretetraethylester,
N-[3-(N'-Morpholino)-propyl-imidobisphosphorsäuretetraethylester,
N-[1-(Cyclohexyl)-ethyl]-imidobisphosphorsäuretetraethylester,
N-Heptyl-imidobisphosphorsäuretetraethylester,
N-Benzyl-imidobisphosphorsäuretetraethylester,
N-Benzyl-imidobisphosphorsäure,
N-[2-(N',N'-Diisopropylamino)ethyl]-imidobisphosphorsäure,
N-[2-(N'-Morpholino)-ethyl]-imidobisphosphorsäure,
N-[2-(2-Pyridyl)-ethyl]-imidobisphosphorsäure,
N-[3-(N'-Morpholino)-propyl]-imidobisphosphorsäure,
N-Cycloheptyl-imidobisphosphorsäure-tetralithium,
N-Cyclohexyl-imidobisphosphorsäure-tetralithium,
N-[1-(Cyclohexyl)-ethyl]-imidobisphosphorsäure-tetralithium,
N-Heptyl-imidobisphosphorsäure-tetralithium,
N-Benzyl-imidobisphosphorsäure-tetralithium,
N-[2-(N,N'-Diisopropylamino)ethyl]-imidobisphosphorsäure-tetralithium,
N-[2-(N,N'-Dibutylamino)ethyl]-imidobisphosphorsäure-tetralithium.

Geeignete physiologisch verträgliche Salze von den Verbindungen der Formel I sind beispielsweise Alkali-, Erdalkali- oder Ammoniumsalze, sowie solche von physiologisch verträglichen, organischen Ammonium- oder Triethylaminbasen.

Gegenstand der Erfindung ist ferner ein Verfahren zur Herstellung von Verbindungen der Formel I 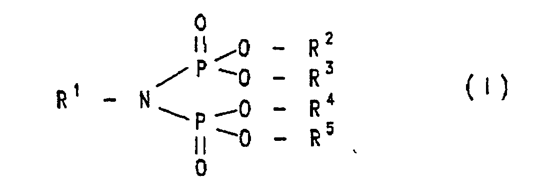 sowie deren physiologisch verträglichen Salze, wobei
- R¹: (C₁-C₁₀)-Alkyl, (C₁-C₈)-Alkanoyl, (C₆-C₁₀)-Aryl oder Het bedeutet, die ein- bis sechsfach substituiert sein können durch
Halogen, -OH, -NH₂, -NH-CO-R⁷, -O-CO-R⁷ und -N-(R⁸)₂,
wobei R⁷ (C₁-C₆)-Alkyl und R⁸ unabhängig voneinander Wasserstoff oder (C₁-C₃)-Alkyl ist, oder
für den Rest der Formel II steht 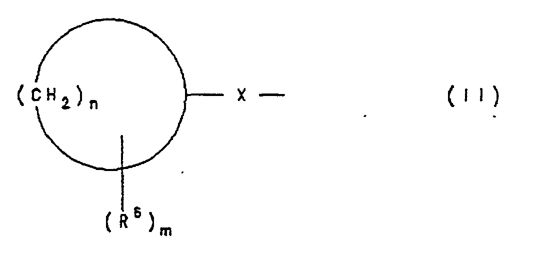 worin
n eine ganze Zahl von 3 bis 10,
m Null bis 3,
R⁶ unabhängig voneinander (C₁-C₆)-Alkyl, das gegebenenfalls ein- bis sechsfach substituiert ist durch Halogen oder -NH-CO-R⁷ mit R⁷ = (C₁-C₆)-Alkyl bedeuten und
X abwesend ist oder (C₁-C₁₀)-Alkyl bedeutet, und
R², R³, R⁴ oder R⁵ unabhängig voneinander für Wasserstoff, (C₁-C₅)-Alkyl, Lithium, Natrium, Kalium oder Si(R)₃, mit R = (C₁-C₅)-Alkyl, stehen, daß dadurch gekennzeichnet ist, daß man
a) Imidobisphosphorigsäurealkylester der Formel III, zugänglich nach dem von L.K. Nikonorova et al., J. Gen. Chem. USSR 46, 1976, 1012 - 1014 beschriebenen Verfahren, 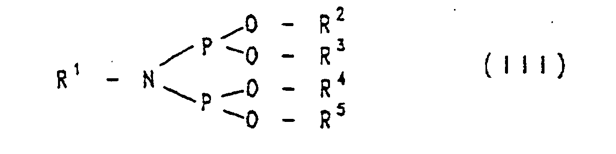 worin
   R¹ die obengenannte Bedeutung hat und
   R², R³, R⁴ und R⁵ für (C₁-C₅)-Alkyl stehen,
   mit einem sauerstoffübertragenden Agenz zu der Verbindung der Formel I umsetzt,
b) gegebenenfalls die so erhaltenen Verbindungen in einem inerten Lösungsmittel mit Hilfe eines Alkyl-halo-silans zu einem Imidobisphosphorsäuretetratrimethylsilylester umsetzt, und
c) anschließend gegebenenfalls hydrolysiert oder mit Hilfe eines basischen Salzes in die entsprechenden Salze überführt.

Beim Reaktionsschritt a) geht man am besten so vor, daß man die Verbindung der Formel III in äquimolaren Mengen oder mit einem bis zu 70 %igen Überschuß Wasserstoffperoxid (35 %) in Alkoholen wie Methanol, Ethanol, n-Propanol, i-Propanol, n-Butanol oder i-Butanol zu den entsprechenden N-substituierten Imidobisphosphorsäuretetraalkylestern der Formel I umsetzt. Die Reaktionstemperaturen liegen dabei zwischen -50 °C und +70 °C, insbesondere bei 30 °C, die Reaktionszeiten liegen zwischen 15 Minuten und 4 Stunden, bevorzugt zwischen 1 und 3 Stunden. Die erhaltenen Rohprodukte können entweder durch Destillation, Kristallisation oder präparative Säulenchromatographie gereinigt werden.

Beim Reaktionsschritt b) geht man am besten so vor, daß man die nach dem Reaktionsschritt a) erhaltenen Verbindungen mit einem vier- bis sechsfachen Überschuß eines Alkylsilans wie beispielsweise Chlor-, Brom- oder Jodtrimethylsilan zur Reaktion bringt. Als Lösungsmittel hierfür eignen sich Toluol, o-,m- oder p-Xylol, Chorbenzol oder Acetonitril, bevorzugt ist Acetonitril, wobei die Lösungsmittel getrocknet eingesetzt werden können. Die Reaktionstemperaturen liegen dabei zwischen 0 °C und 70 °C, bevorzugt bei 20 °C bis 30 °C. Die Reaktionszeiten liegen zwischen 1 Stunde und 24 Stunden, bevorzugt zwischen 4 Stunden und 18 Stunden. Anschließend werden das jeweilige Lösungsmittel und der Überschuß an Silan im Vakuum entfernt. Als Rückstand wird das gewünschte Produkt der Formel I erhalten.

Beim Reaktionsschritt c) geht man am besten so vor, daß man die nach dem Reaktionsschritt b) erhaltenen Verbindungen in einem Lösungsmittel wie beispielsweise ein Alkohol, bevorzugt Methanol, Ethanol, n-Propanol, i-Propanol, n-Butanol oder i-Butanol, besonders bevorzugt Methanol, Ethanol oder i-Propano löst und bis zur Kristallisation der Verbindung der Formel I bei einer Temperatur von 10 °C bis 30 °C, bevorzugt bei 25 °C stehen läßt, oder man gibt 4 Moläquivalente bis 100 Moläquivalente, bevorzugt 4 Moläquivalente bis 20 Moläquivalente Wasser zu der Lösung bei einer Temperatur von 10 °C bis 30 °C, bevorzugt bei 25 °C und läßt bis zur Kristallisation der Verbindung stehen. Die so erhaltenen Verbindungen werden anschließend in einem inerten Lösungsmittel wie beispielsweise n-Hexan, n-Heptan, Methylcyclohexan, Petrolether (40 - 60 °C), bevorzugt n-Hexan oder Petrolether (40 - 60 °C) aufgeschlämmt und filtriert oder man löst die nach dem Reaktionsschritt b) erhaltenen Verbindungen direkt in einem Lösungsmittel wie beispielsweise n-Hexan, n-Heptan, Methylcyclohexan oder Petrolether (40 - 60 °C), bevorzugt n Hexan oder Petrolether (40 - 60 °C). Zu dieser Lösung gibt man 4 bis 10 Moläquivalente, bevorzugt 4 Moläquivalente eines Alkalialkoholats wie beispielsweise Lithiummethanolat, Natriummethanolat, Kaliummethanolat, bevorzugt Lithiummethanolat, Natriummethanolat oder Kaliummethanolat, besonders bevorzugt Lithiummethanolat oder Natriummethanolat, gelöst in einem Alkohol wie beispielsweise Methanol oder Ethanol, bevorzugt Methanol. Die Reaktionstemperaturen liegen zwischen -10 °C und 40 °C, bevorzugt zwischen -5°C und 30 °C. Die Reaktionszeiten liegen zwischen 1 Stunde und 20 Stunden, bevorzugt zwischen 1 Stunde und 14 Stunden. Die Verbindungen der Formel I kristallisieren in der Regel aus der Reaktionslösung hochrein aus.

Gegenstand der Erfindung sind auch Arzneimittel, die mindestens eine Verbindung der Formel I und/oder mindestens eines von deren physiologisch verträglichen Salze enthalten, neben pharmazeutisch geeigneten und physiologisch verträglichen Hilfs- und Trägerstoffen, Verdünnungsmitteln und/oder anderen Wirkstoffen.

Ein weiterer Gegenstand der Erfindung ist die Verwendung der Verbindungen dei Formel I sowie deren physiologisch verträglichen Salze zur Prophylaxe und Behandlung von Osteoporose und Hypercalcemia, bevorzugt Osteoporose.

Die erfindungsgemäßen Arzneimittel können intravenös, intramuskulär, intraperitoneal, subcutan, intraartikulär, periartikulär, rektal oder oral verabreicht werden.

Die Herstellung der erfindungsgemäßen Arzneimittel zur Behandlung degenerativer Gelenkserkrankungen erfolgt, indem mindestens eine Verbindung der Formel I und/oder eines von deren physiologisch verträglichen Salze gegebenenfalls mit weiteren Hilfs- und/oder Trägerstoffen in eine geeignete Darreichungsform gebracht wird. Die Hilfs- und Trägerstoffe stammen aus der Gruppe der Trägermittel, Konservierungsstoffe und anderer üblicher Hilfsstoffe.

Zum Beispiel können für orale Darreichungsformen Hilfsstoffe wie Stärken, z.B. Kartoffel-, Mais- oder Weizenstärke, Cellulose bzw. deren Derivate, insbesondere mikrokristalline Cellulose, Siliziumdioxid, verschiedene Zucker wie Milchzucker, Magnesiumcarbonat und/oder Calciumphosphate verwendet werden. Weiterhin ist es vorteilhaft, den oralen Darreichungsformen Hilfsstoffe zuzusetzen, die die Verträglichkeit der Medikamente verbessern, wie z.B. Schleimbildner und Harze. Zwecks besserer Verträglichkeit können die Medikamente auch in Form von magensaftunlöslichen Kapseln verabreicht werden. Darüber hinaus kann es vorteilhaft sein, der Darreichungsform, bzw. einer Komponente eines Kombinationspräparates, ein Retardierungsmittel zuzusetzen, gegebenenfalls in Form von permeablen Membranen, wie z.B. solche auf Cellulose- oder Polystrolharzbasis oder lonenaustauscher.

Die anzuwendende Dosierung der erfindungsgemäßen Arzneimittel ist abhängig von verschiedenen Faktoren wie Darreichungsform des Medikamentes und Zustand, Gewicht und Schwere der Erkrankung des Patienten. Eine Tagesdosis von ca. 5000 mg der erfindungsgemäßen Arzneimittel sollte jedoch nur kurzzeitig überschritten werden. Bevorzugt sind ca. 10 bis 2500 mg als Tagesdosis für einen Menschen von ca. 70 kg Körpergewicht. Die Verabreichung der Tagesdosis der erfindungsgemäßen Arzneimittel kann in Form einer einzelnen Verabreichung oder in mehreren kleinen Dosen erfolgen. Bevorzugt ist die Verabreichung in 3 bis 8 Dosen pro Tag.

Die Wirksamkeit der erfindungsgemäßen Verbindungen der Formel I wird in vitro in dem folgenden Versuch nachgewiesen:

Hydroxylapatit-Adsorptions-Test (Shinoda et al., Calcf. Tissue Int. 1983, Vol. 35, 87 - 99):

65 mg Hydroxylapatit wurden in 200 ml 0,01 M Barbital Puffer (pH 7,0), der 0,115 M KCI enthält, suspendiert und bei 37 °C 24 Stunden gerührt. Anschließend wurden die zu testenden Substanzen (0,075 mol pro mol Hydroxylapatit) zu der equilibrierten Suspension zugefügt und weitere 2 Stunder gerührt. Die Suspension wurde durch einen Filter (0,45 *µ*m Porengröße) gegeben, das Hydoxylapatit vom Filter gekratzt und anschließend wieder in 75 ml des gleichen Barbital Puffers suspendiert und bei 37 °C gerührt. Nach 1 Stunde wurde die Calcium-Konzentration des Puffers mit einem automatischen Calcium-Konzentrations-Analysator (Hitachi Model 7050) bestimmt. Aus diesem Wert wurde die Fähigkeit die Calcium-Freisetzung zu hemmen für die getesteten Verbindungen berechnet. Die Bestimmung wurde für jede Verbindung 3 x wiederholt. Als Standard ist der Wert für Methandiphosphonsäure angegeben.

Die Ergebnisse sind in Tabelle 1 zusammengestellt.

**Tabelle 1**

| Verbindung | Calcium-Konzentration [mg/dl] |
|---|---|
| Kontrolle | 0,663 ± 0,008 |
| Methandiphosphonsäure | 0,537 ± 0,010 * |
| Beispiel 18 | 0,603 ± 0,006 * |
| Beispiel 20 | 0,607 ± 0,006 * |
| Beispiel 21 | 0,553 ± 0,010 * |
| Beispiel 22 | 0,513 ± 0,010 * |

| | |
|---|---|
| * signifikante Differenz 1 % | |

Alle getesteten Verbindungen hemmten die Calcium-Freisetzung aus Hydroxylapatit signifikant.

### Beispiele:

### 1) N-Cycloheptyl-imidobisphosphorsäuretetraethylester

### a) N-Cycloheptyl-imidobisphosphorigsäuretetraethylester

8.0 g (71 mMol) Cycloheptylamin und 14.8 g (142 mMol) Triethylamin werden in 80 ml Dichlormethan bei 0°C vorgelegt. Hierzu tropft man 22.0 g (142 mMol) Phosphorigsäurediethylestermonochlorid, gelöst in 20 ml Dichlormethan bei 0°C. Nach beendeter Zugabe läßt man die Reaktionslösung auf Raumtemperatur kommen und rührt über Nacht. Anschließend wird das gebildete Salz abfiltriert. Dabei isoliert man 16.5 g (85 % d.Th.) Triethylaminhydrochlorid. Um weiteres Hydrochlorid zu isolieren wird die Lösung bei -30°C für 4 Stunden gekühlt. Erneut ausgefallenes Hydrochlorid wird über Perlit abgesaugt und die Lösung eingeengt (12 mbar). Man erhält auf diese Weise 20.7 g N-Cycloheptyl-imidobisphosphorigsäuretetraethylester (³¹P-NMR (CDCl₃): δ = 146.7 ppm) als Rohprodukt. Dieses Rohprodukt wird direkt wie unter b) beschrieben weiter umgesetzt.

### b) N-Cycloheptyl-imidobisphosphorsäuretetraethylester

20.7 g (58 mMol) Rohprodukt aus a) werden in 150 ml Methanol gelöst und zu einer Lösung aus 22 g (ca. 50 %iger Überschuß) 35 %ige Wasserstoffperoxidlösung in 100 ml Methanol getropft, so daß die Reaktionstemperatur 30°C nicht übersteigt. Nach dem beendeten Zutropfen rührt man 1 Stunde nach und entfernt 200 ml Methanol im Vakuum (12 mbar, nicht bis zur Trockne). Der Rückstand wird in 200 ml Dichlormethan aufgenommen und 3 mal mit je 80 ml Wasser extrahiert. Die Dichlormethanphase wird über Natriumsulfat getrocknet und eingedampft (12 mbar). Man erhält 18 g Rohprodukt als Öl. Das Rohprodukt wird destillativ gereinigt.

C₁₅H₃₃NO₆P₂ (385.4), Ausbeute: 10.0 g (44.1 %), Sdp. 137-139°C/0.06 mbar; MS: (FAB) 386 (70) M⁺, 290 (30) [M-Cycloheptyl]⁺; EA: ber. C = 46.8, H = 8.6, N = 3.6, gef. C = 46.9, H = 8.4, N = 3.7 %; ¹H-NMR (CDCl₃): δ = 1.35 (t, ³J_{HH}=7.0 Hz, 12 H), 1.40-1.50 (m, 2H), 1.50-1.60 (m, 4H), 1.67-1.79 (m, 2H), 1.84-1.96 (m, 2H), 2.20-2.35 (m, 2H), 3.58 (m, 1H), 4.13 (m, 8H) ppm; ¹³C-NMR (CDCl₃): δ = 16.0 (CH₃(OEt)), 25.5 (CH₂), 26.9 (CH₂), 34.8 (CH₂), 61.9 (CH), 62.8 (CH₂(OEt)) ppm; ³¹P-NMR (CDCl₃): δ = 3.55 ppm.

Die nachfolgenden Verbindungen der Beispiele 2 - 10 wurden in analoger Weise hergestellt. Bei Verbindungen, die nicht destillativ gereinigt werden konnten, erfolgte die Reinigung durch präparative Säulenchromatographie.

### 2) N-Butyl-imidobisphosphorsäuretetraethylester

C₁₂H₂₉NO₆P₂ (345.3), Ausbeute: 15.5 g (84.7 %), Sdp. 120-123°C/0.1 mbar; MS: (FAB) 345 (70) M⁺; EA: ber. C = 41.7, H = 8.4, N = 4.1, gef. C = 41.7, H = 8.4, N = 4.8; ¹H-NMR (CDCl₃): δ = 0.93 (t, ³J_{HH} = 7.3 Hz, 3H), 1.30 (m, 2H), 1.35 (t, ³J_{HH} = 7.0 Hz, 12H), 1.69 (m, 2H), 3.31 (m, 2H), 4.15 (m, 8H) ppm; ¹³C-NMR (CDCl₃): δ = 13.60 (CH₃), 15.96 (CH₃(OEt)), 19.81 (CH₂),32.95 (CH₂), 47.57 (CH₂), 63.08 (CH₂(OEt)) ppm; ³¹P-NMR (CDCl₃): δ = 3.98 ppm.

### 3) N-[2-(N',N'-Diisopropylamino)-ethyl]-imidobisphosphorsäuretetraethylester

Das Rohprodukt wurde über eine Aceton/Kieselgelsäule gereinigt. C₁₆H₃₈N₂O₆P₂ (416.5), Ausbeute: 14.9 g (71.2%), MS: (FAB) 416 (60) M⁺; ¹H-NMR (CDCl₃): δ = 1.03 (d, ³J_{HH} = 6.4 Hz, 12H), 1.36 (t, ³J_{HH} = 7.0 Hz, 12H), 2.67 (m, 2H), 2.98 (m, ³J_{HH} = 6.4 Hz, 2H), 3.24 (m, 2H), 4.15 (m, 8H) ppm; ¹³C-NMR (CDCl₃): **δ** = 16.00 (CH₃(OEt)), 20.66 (CH₃), 45.69 (CH₂), 48.64 (CH₂), 49.62 (CH), 63.14 (CH₂(OEt)) ppm; ³¹P-NMR (CDCl₃): δ = 4.33 ppm.

### 4) N-[2-(N'-Morpholino)-ethyl]-imidobisphosphorsäuretetraethylester

Das Rohprodukt wurde über eine Essigester/Ethanol = 1/1 Kieselgelsäule gereinigt.
C₁₄H₃₂N₂O₇P₂ (402.4), Ausbeute: 11.3 g (40.1 %); MS (FAB): 402 (70) M⁺; ¹H-NMR (CDCl₃): δ = 1.36 (t, ³J_{HH} = 7.0 Hz, 12H), 2.52 (m, 4H), 2.63 (m, 2H), 3.48 (m, 2H), 3.68 (m, 4H), 4.16 (m, 8H) ppm; ³¹P-NMR (CDCl₃): δ = 4.54 ppm.

### 5) N-[2-(2-Pyridyl)-ethyl]-imidobisphosphorsäuretetraethylester

Das Rohprodukt wurde über eine Essigester/Ethanol = 1/1 Kieselgelsäule gereinigt.
C₁₅H₂₈N₂O₆P₂ (394.4), Ausbeute: 14.7 g (53.3 %); ¹H-NMR (CDCl₃): δ = 1.37 (t, ³J_{HH} = 7.0 Hz, 12H), 3.22 (m, 2H), 3.73 (m, 2H), 4.18 (m, 8H), 7.12 (m, 1H), 7.23 (m, 1H), 7.59 (m, 1H), 8.52 (m, 1H) ppm; ³¹P-NMR (CDCl₃): δ = 4.24 ppm.

### 6) N-[3-(N'-Morpholino)-propyl]-imidobisphosphorsäuretetraethylester

Das Rohprodukt wurde über eine Essigester/Ethanol = 1/1 Kieselgelsäule gereinigt.
C₁₅H₃₄N₂O₇P₂ (416.4), Ausbeute: 14.9 g (54.5 %); ¹ H-NMR (CDCl₃): δ = 1.35 (t, ³J_{HH} = 7.0 Hz,12H), 1.91 (m, 2H), 2.35 (t, 2H), 2.43 (m, 4H), 3.39 (m, 2H), 3.70 (m, 4H), 4.14 (m, 8H) ppm; ³¹P-NMR (CDCl₃): δ = 4.46 ppm.

### 7) N-Cyclohexyl-imidobisphosphorsäuretetraethylester

C₁₄H₃₁NO₆P₂ (371.4), Ausbeute: 18 g (69.4 %), Sdp.: 136°C/0.1 mbar; ¹H-NMR (CDCl₃): δ = 1.20 (m, 4H), 1.34 (t, ³J_{HH} = 7.0 Hz, 12H), 1.80 (m, 4H), 2.16 (m, 2H), 3.50 (m, ³J_{PH} = 19.1 Hz, 1H), 4.13 (m, 8H) ppm; ³¹P-NMR (CDCl₃): δ = 4.08 ppm.

### 8) N-[1-(Cyclohexyl)-ethyl]-imidobisphosphorsäuretetraethylester

C₁₆H₃₅NO₆P₂ (399.3), Sdp. 142°C/0.1 mbar, Ausbeute: 19.7 g (69.8 %); ¹H-NMR (CDCl₃): δ = 1.19 (m, 4H), 1.34 (t, ³J_{HH} = 7.0 Hz, 12H), 1.42 (d, 3H), 1.69 (m, 4H), 2.06 (m, 2H), 3.51 (m, 1H), 4.05 (m, 1H), 4.13 (m, 8H) ppm; ³¹P-NMR (CDCl₃): **δ** = 4.75 ppm.

### 9) N-Heptyl-imidobisphosphorsäuretetraethylester

C₁₅H₃₅NO₆P₂ (387.4), Ausbeute: 29 g (56 %), Sdp.: 147-150°C/0.15 mbar; ¹H-NMR (CDCl₃): δ = 0.88 (t, 3H), 1.28 (m, 8H), 1.35 (t, ³J_{HH} = 7.0Hz, 12H), 1.69 (m, 2H), 3.30 (m, 2H), 4.15 (m, 8H) ppm; ³¹P-NMR (CDCl₃): δ = 4.24 ppm.

### 10) N-Benzyl-imidobisphosphorsäuretetraethylester

Bei der Aufarbeitung wurden die Leichtsieder bei 130°C/10⁻³ mbar entfernt und so N-Benzyl-imidobisphosphorsäuretetraethylester in reiner Form erhalten. C₁₅H₂₇NO₆P₂ (379.3), Ausbeute: 17.1 g (86.3 %); ¹H-NMR (CDCl₃): δ = 1.21 (t, ³J_{HH} = 7.0 Hz, 12H), 4.08 (m, 8H), 4.56 (t, ³J_{PH} = 12.5 Hz, 2H), 7.29 (m, 3H), 7.53 (m, 2H) ppm; ³¹P-NMR (CDCl₃): δ = 3.98 ppm.

### 11) N-[2-(N',N'-Diisopropylamino)-ethyl]-imidobisphosphorsäure

### a) N-[2-(N',N'-Diisopropylamino)-ethyl]-imidobisphosphorsäure-tetratrimethylsilylester

2.2 g (5.3 mMol) N-[2-(N',N'-Diisopropylamino)-ethyl]imidobisphosphorsäuretetraethylester werden in 30 ml wasserfreiem Acetonitril gelöst. Zu dieser Lösung gibt man innerhalb von einer Stunde bei 25°C 4.9 g = 4.2 ml (31.8 mMol) Bromtrimethylsilan. Die Reaktion wird anschließend 18 Stunden bei dieser Temperatur gerührt. Am Ende der Reaktion werden das Lösungsmittel und überschüssiges Bromtrimethylsilan bei 0.06 mbar/40°C abgezogen. Der Rückstand ist wachsartig.

### b) N-[2-(N',N'-Diisopropylamino)-ethyl]-imidobisphosphorsäure

1.5g (0.76 mMol) des Silylester aus a) werden in 30 ml i-Propanol gelöst. Hierzu tropft man 2.1 ml (120 mMol) Wasser und rührt 4 Stunden bei 25°C. Anschließend setzt man 20 ml Essigester zu und läßt ca. 1 Woche zur Kristallisation stehen. Die ausgefallenen Kristalle werden unter Schutzgas abgesaugt und bei 0.06 mbar/ 25°C getrocknet.

C₈H₂₂N₂O₆P₂ (304.1), Ausbeute: 420 mg (26.3 %), Schmp.: 143°C; MS (FAB): 304 (90) M⁺; EA (3H₂O): ber. C = 26.8, H = 7.8, N = 7.8, gef. C = 27.1, H = 6.8, N = 7.7; ¹H-NMR (D₂O): δ = 1.36 (dd, ³J_{HH} = 6.6 Hz, 12H), 3.29 (t, 2H), 3.62 (tt, ³J_{PH} = 13.5 Hz, 2H), 3.73 (m, 2H) ppm; ³¹P-NMR (D₂O): δ = 3.88 ppm.

### 12) N-[2-(N'-Morpholino)-ethyl]-imidobisphosphorsäure

### a) N-[2-(N'-Morpholino)-ethyl]-imidobisphosphorsäuretetratrimethylsilylester

2.12 g (5.26 mMol) N-[2-(N'-Morpholino)-ethyl]imidobisphosphorsäuretetraethylester werden wie unter 11a beschrieben umgesetzt.

### b) N-[2-(N'-Morpholino)-ethyl]-imidobisphosphorsäure

Zu dem unter a) erhaltenen Rückstand werden 30 ml Methanol gegeben und 2 Stunden bei 25°C gerührt. Dabei kristallisiert das Produkt als farbloses Pulver aus. Die Substanz wird an Luft abfiltriert, mit Petrolether (40-60°C) nachgewaschen und an Luft getrocknet.

C₆H₁₆N₂O₇P₂ (290.2), Ausbeute: 990 mg (64.7 %), Schmp.: 203°C; MS (FAB): 290 (95) M⁺; EA: ber. C = 24.8, H = 5.5, N = 9.4, gef. C = 24.9, H = 5.3, N = 9.4; ¹H-NMR (D₂O): δ = 3.20 (m, 2H), 3.36 (t, 2H), 3.60 (m, 2H), 3.62 (tt, ³J_{PH} = 13.5 Hz, 2H), 3.81 (m, 2H), 4.12 (m, 2H) ppm; ³¹P-NMR. δ = 3.32 ppm.

### 13) N-[2-(2-Pyridyl)-ethyl]-imidobisphosphorsäure

### a) N-[2-(2-Pyridyl)-ethyl]-imidobisphosphorsäuretetratrimethylsilylester

1.61 g (5.26 mMol) N-[2-(2-Pyridyl)-ethyl]-imidobisphosphorsäuretetraethylester werden wie unter 11a) beschrieben umgesetzt.

### b) N-[2-(2-Pyridyl)-ethyl]-imidobisphosphorsäure

Zu dem unter a) erhaltenen Rückstand werden 30 ml Methanol gegeben und 2 Stunden bei 25°C gerührt, dabei kristallisiert das Produkt als farbloses Pulver aus. Die Substanz wird an Luft abfiltriert, mit Petrolether (40-60°C) gewaschen und an Luft getrocknet.

C₇H₁₂N₂O₆P₂ (282.1), Ausbeute: 650 mg (46.6 %), Schmp.: 186°C; MS (FAB): 282 (90) M⁺; EA (1/2 H₂O) ber. C = 28.8, H = 4.5, N = 9.6, gef. C = 28.8, H = 4.3, N = 9.4; ¹H-NMR (D₂O): δ = 3.39 (t, 2H), 3.65 (tt, ³J_{PH} = 13.5 Hz, 2H), 7.88 (m, 1H), 7.96 (m, 1H), 8.47 (m, 1H), 8.63 (m, 1H) ppm; ³¹P-NMR (D₂O): δ = 3.26 ppm.

### 14) N-[3-(N'-Morpholino)-propyl]-imidobisphosphorsäure

### a) N-[3-(N'-Morpholino)-propyl]-imidobisphosphorsäuretetratrimethylsilylester

2.19 g (5.3 mMol) N-[3-(N'-(Morpholino)-propyl]-imidobisphosphorsäuretetraethylester werden wie unter 11a) beschrieben umgesetzt,

### b) N-[3-(N'-Morpholino)-propyl]-imidobisphosphorsäure

Zu dem unter a) erhaltenen Rückstand werden 20 ml Methanol gegeben und 2 Stunden bei 25°C gerührt, dabei kristallisiert das Produkt als farbloses Pulver aus. Die Substanz wird abfiltriert, mit 10 ml Methanol und anschließend 10 ml Petrolether (40-60°C) gewaschen und an Luft getrocknet.

C₇H₁₈N₂O₇P₂ (304.2), Ausbeute: 450 mg (31.3 %), Schmp.: 75°C; MS (FAB) 304 (60) M⁺; ¹H-NMR (D₂O): δ = 2.09 (m, 2H), 3.14 (m, 2H), 3.26 (m, 2H), 3.39 (tt, ³J_{PH} = 13.5 Hz, 2H), 3.53 (m, 2H), 3.81 (m, 2H), 4.12 (m, 2H) ppm; ³¹P-NMR (D₂O): δ = 3.72 ppm.

### 15) N-Cycloheptyl-imidobisphosphorsäure-tetralithium-salz

### a) N-Cycloheptyl-imidobisphosphorsäuretetratrimethylsilylester

2.03 g (5.26 mMol) N-Cyclohepthyl-imidobisphosphorsäuretetraethylester werden wie unter 11a) beschrieben umgesetzt.

### b) N-Cycloheptyl-imidobisphosphorsäure-tetralithium-salz

Die Umsetzung erfolgt wie unter 17b) beschrieben.

C₇H₁₃NO₆P₂Li₄ (296.9), Ausbeute: 1.7 g (77 %), Schmp.: > 230°C; EA (4 CH₃OH): ber. C = 31.0, H = 6.8, N = 3.3, gef. C = 31.8, H = 6.2, N = 3.8; ¹H-NMR: δ = 1.34-1.68 (m, 8H), 1.82-2.12 (m, 4H), 3.41 (m, ³J_{PH} = 20.8 Hz, 1H) ppm; ³¹P-NMR (D₂O): δ = 8.01 ppm.

### 16) N-Cyclohexyl-imidobisphosphorsäure-tetralithium-salz

### a) N-Cyclohexyl-imidobisphosphorsäuretetratrimethylsilylester

1.95 g (5.26 mMol) N-Cyclohexyl-imidobisphosphorsäuretetraethylester werden wie unter 11a) beschrieben umgesetzt.

### b) N-Cyclohexyl-imidobisphosphorsäure-tetralithium-salz

Die Umsetzung erfolgt wie unter 17b) beschrieben.

C₆H₁₁NO₆P₂Li₄ (282.8), Ausbeute: 1.8 g (90 %), Schmp.: > 230°C; EA (3 CH₃OH): ber. C = 28.5, H = 6.1, N = 3.7, gef. C = 28.8, H = 5.7, N = 3.8; ¹H-NMR (D₂O): δ = 1.00-1.33 (m, 3H), 1.53 (m, 1H), 1.74 (m, 4H), 1.94 (m, 2H), 3.27 (m, ³J_{PH} = 21.0 Hz, 1H) ppm; ³¹P-NMR (D₂O): δ = 7.96 ppm.

### 17) N-[1-(Cyclohexyl)-ethyl]-imidobisphosphorsäure-tetralithium-salz

### a) N-[1-(Cyclohexyl)-ethyl]-imidobisphosphorsäuretetratrimethylsilylester

1.05 g (2.63 mMol) N-[1-(Cyclohexyl)-ethyl]-imidobisphosphorsäuretetraethylester werden wie unter 11a) beschrieben umgesetzt.

### b) N-[1-(Cyclohexyl)-ethyl]-imidobisphosphorsäure-tetralithium-salz

Der Rückstand aus a) wird in 15 ml Petrolether (40-60°C) aufgenommen und auf 0°C gekühlt. Zu dieser Lösung gibt man 0.4 g Lithiummethylat gelöst in 3 ml Methanol, rührt eine weitere Stunde bei 0°C und anschließend 12 Stunden bei 25°C. Es bildet sich ein gelartiger Niederschlag. Der Petrolether wird bei 0.06 mbar/ 25°C abdestilliert, der Rückstand in 15 ml Methanol aufgenommen und eine weitere Stunde bei 25°C gerührt. Nach Filtration des Niederschlags setzt man erneut 15 ml Petrolether (40-60°C) zu, rührt aus und filtriert erneut.

Dabei erhält man ein farbloses Pulver, welches bei 0.06 mbar/ 25°C getrocknet wird.

C₈H₁₅NO₆P₂Li₄ (310.8), Ausbeute: 890 mg (90 %), Schmp.: > 230°C; EA (2 CH₃OH). ber. C = 32.0, H = 6.2, H = 3.7, gef. C = 32.6, H = 6.0, N = 3.8; ¹H-NMR (D₂O): δ = 0.8 (m, 2H), 1.17 (m, 2H), 1.28 (d, 3H), 1.55-1.95 (m, 6H), 2.21 (m, 1H), 3.13 (m, ³J_{PH} = 20.2 Hz, 1H) ppm; ³¹P-NMR (D₂O): δ = 8.04 ppm;.

### 18) N-Heptyl-imidobisphosphorsäure-tetralithium-salz

### a) N-Heptyl-imidobisphosphorsäuretetratrimethylsilylester

1.02 g (2.63 mMol) N-Heptyl-imidobisphosphorsäuretetraethylester werden wie unter 11a) beschrieben umgesetzt.

### b) N-Heptyl-imidobisphosphorsäure-tetralithium-salz

Die Umsetzung erfolgt wie unter 17b) beschrieben. Das Produkt wird zur Kristallisation in 15 ml Methanol aufgenommen, filtriert und bei 0.06 mbar/25°C getrocknet.

C₇H₁₅NO₆P₂Li₄ (298.9), Ausbeute: 450 mg (52 %), Schmp.: > 230°C; EA (CH₃OH): ber. C = 29.0, H = 5.8, N = 4.2, gef. C = 29.2, H = 5.9, N = 3.8; ¹H-NMR (D₂O): δ = 0.87 (t, 3H), 1.12-1.39 (m, 8H), 1.62 (m, 2H), 3.00 (m, ³J_{PH} = 13.9 Hz, 2H) ppm; ³¹P-NMR (D₂O): δ = 8.01 ppm.

### 19) N-Benzyl-imidobisphosphorsäure-tetranatrium-salz

### a) N-Benzyl-imidobisphosphorsäuretetratrimethylsilylester

1.05 g (2.63 mMol) N-Benzyl-imidobisphophorsäuretetraethylester werden wie unter 11a) beschrieben umgesetzt.

### b) N-Benzyl-imidobisphosphorsäure-tetranatrium-salz

Der Rückstand aus a) wird in 10 ml Petrolether (40-60°C) aufgenommen und auf 0°C gekühlt. Zu dieser Lösung gibt man 0.25 g Natriummethylat gelöst in 3 ml Methanol innerhalb von 10 Minuten. Direkt nach der Zugabe bildet sich ein Zweiphasensystem, welches innerhalb von 30 Minuten homogenisiert. Es bildet sich innerhalb von 2-3 Stunden ein wachsartiges Produkt, welches nach 2 Tagen kristallin wird. Dieses Produkt wird mit Hilfe einer Schlenkfritte abgesaugt und zweimal mit je 20 ml Aceton gewaschen. Hierbei erhält man ein farbloses kristallines Produkt, welches bei 0.06 mbar/ 25°C getrocknet wird.

C₇H₇NO₆P₂Na₄ (354.7), Ausbeute: 770 mg (78.5 %), Schmp.: > 230°C; ¹H-NMR (D₂O): δ = 4.40 (t, ³J_{PH} = 12.5 Hz, 2H), 7.20-7.65 (m, 5H) ppm; ³¹P-NMR (D₂O): δ = 7.60 ppm.

### 20) N-Benzyl-imidobisphosphorsäure

Die Herstellung erfolgt analog dem unter 1) und 11) beschriebenen Verfahren.

C₇H₁₁NO₆P₂ (267.1), Ausbeute: 526 mg (75 %); ³¹P-NMR (D₂O): δ = 3.86 ppm.

### 21) N-[2-(N',N-Diisopropylamino)ethyl]-imidobisphosphorsäuretetralithium-salz

Die Herstellung erfolgt analog dem unter 1) und 17) beschriebenen Verfahren.

C₈H₁₈N₂O₆P₂Li₄ (327.2), Ausbeute: 631 mg (80 %); ³¹P-NMR (D₂O): δ = 7.13 ppm.

### 22) N-[2-(N',N-Dibutylamino)ethyl]-imidobisphosphorsäuretetralithium-salz

Die Herstellung erfolgt analog dem unter 1) und 17) beschriebenen Verfahren.

C₁₀H₂₂N₂O₆P₂Li₄ (355.9), Ausbeute: 673 mg (72 %); ³¹P-NMR (D₂O): δ = 6.95 ppm.

## Patentansprüche

1. Verbindungen der Formel I, 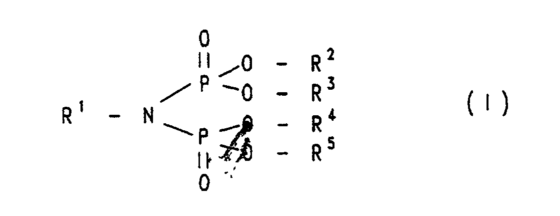 sowie deren physiologisch verträgliche Salze, worin
R¹ für (C₁-C₁₀)-Alkyl, (C₁-C₈)-Alkanoyl, (C₆-C₁₀)-Aryl oder Het steht, die einfach bis sechsfach substituiert sein können durch Halogen, -OH, -NH₂, -NH-CO-R⁷, -O-CO-R⁷ und -N(R⁸)₂,
worin R⁷ für (C₁-C₆)-Alkyl und R⁸ unabhängig voneinander für Wasserstoff oder (C₁-C₃)-Alkyl steht, und
Het für einen monocyclischen 5- bis 6-gliedrigen oder bicyclischen 8- bis 10-gliedrigen gesättigten, ungesättigten oder teilweise ungesättigten heterocyclischen Rest mit 1 bis 3 gleichen oder verschiedenen Heteroatomen aus der Reihe Stickstoff, Sauerstoff und Schwefel steht,
oder für den Rest der Formel II 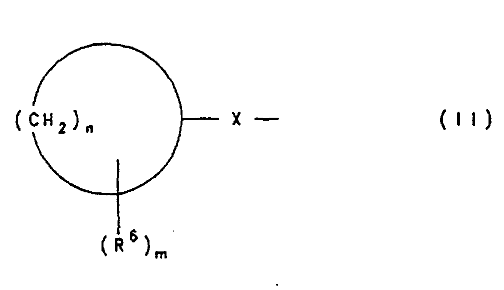 steht, worin
n eine ganze Zahl von 3 bis 10,
m Null bis 3,
R⁸ unabhängig voneinander (C₁-C₆)-Alkyl, das einfach bis sechsfach substituiert sein kann durch Halogen oder -NH-CO-R⁷, mit R⁷ = (C₁-C₆)-Alkyl, bedeuten, und
X abwesend ist oder (C₁-C₁₀)-Alkyl bedeutet, und
R², R³, R⁴ oder R⁵ unabhängig voneinander für Wasserstoff, (C₁-C₅)-Alkyl, Lithium, Natrium, Kalium oder Si(R)₃, mit R = (C₁-C₅)-Alkyl, stehen,
wobei ausgeschlossen sind die Verbindungen der Formel I mit
R¹ = Methyl und R² - R⁵ gleichzeitig = Methyl, Ethyl, Propyl, Isopropyl, Butyl oder Isobutyl oder R² und R³ = Ethyl oder Isopropyl und R⁴ und R⁵ = Methyl;
R¹ = Ethyl und R² - R⁵ gleichzeitig = Ethyl, Propyl, Isopropyl, Butyl oder Isobutyl oder R² und R³ = Ethyl und R⁴ und R⁵ = Methyl;
R¹ = Isopropyl und R² und R³ = Isopropyl und R⁴ und R⁵ = Methyl;
R¹ = Butyl und R² - R⁵ gleichzeitig = Ethyl, Isopropyl oder Butyl;
R¹ = Isobutyl und R² - R⁵ gleichzeitig = Ethyl;
R¹ = Pentyl und R² - R⁵ gleichzeitig = Ethyl, Propyl oder Butyl;
R¹ = 1-tert. Butyl-1,1'-dimethyl-propyl und R² - R⁵ gleichzeitig = Ethyl;
R¹ = Dodecyl und R² - R⁵ gleichzeitig = Butyl;
R¹ = Phenyl und R² - R⁵ gleichzeitig = Propyl; und
R¹ = Cyclohexyl und R² - R⁵ gleichzeitig = Ethyl.

2. Verbindungen der Formel I gemäß Anspruch 1 sowie deren physiologisch verträgliche Salze, **dadurch gekennzeichnet, daß**
R¹ für (C₁-C₁₀)-Alkyl, (C₁-C₈)-Alkanoyl, (C₆-C₁₀)-Aryl oder Het steht, die einfach bis sechsfach substituiert sein können durch Halogen, -OH, -NH₂, -NH-CO-R⁷, -O-CO-R⁷ und -N(R⁸)₂,
worin R⁷ für (C₁-C₆)-Alkyl und R⁸ unabhängig voneinander für Wasserstoff oder (C₁-C₃)-Alkyl steht, und
Het ein oder zwei Stickstoffatome enthält,
oder für den Rest der Formel II 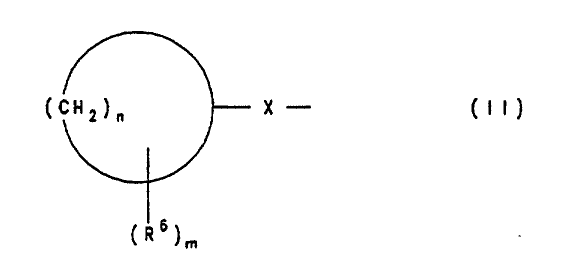 steht, worin
n eine ganze Zahl von 3 bis 10,
m 1,
R⁶ (C₁-C₆)-Alkyl, das einfach bis sechsfach substituiert sein kann durch Halogen oder -NH-CO-R⁷, mit R⁷ = (C₁-C₆)-Alkyl, bedeuten, und
X abwesend ist oder (C₁-C₁₀)-Alkyl bedeutet, und
R² und R³, R⁴ und R⁵ für Wasserstoff, Lithium, Natrium oder (C₁-C₅)-Alkyl stehen,
wobei ausgeschlossen sind Verbindungen der Formel I mit
R¹ = Methyl und R² - R⁵ gleichzeitig = Methyl, Ethyl, Propyl, Isopropyl, Butyl oder Isobutyl oder R² und R³ = Ethyl oder Isopropyl und R⁴ und R⁵ = Methyl;
R¹ = Ethyl und R² - R⁵ gleichzeitig = Ethyl, Propyl, Isopropyl, Butyl oder Isobutyl oder R² und R³ = Ethyl und R⁴ und R⁵ = Methyl;
R¹ = Isopropyl und R² und R³ = Isopropyl und R⁴ und R⁵ = Methyl;
R¹ = Butyl und R² - R⁵ gleichzeitig = Ethyl, Isopropyl oder Butyl;
R¹ = Isobutyl und R² - R⁵ gleichzeitig = Ethyl;
R¹ = Pentyl und R² - R⁵ gleichzeitig = Ethyl, Propyl oder Butyl;
R¹ = 1-tert. Butyl-1,1'-dimethyl-propyl und R² - R⁵ gleichzeitig = Ethyl;
R¹ = Phenyl und R² - R⁵ gleichzeitig = Propyl; und
R¹ = Cyclohexyl und R² - R⁵ gleichzeitig = Ethyl.

3. Verbindungen der Formel I gemäß Anspruch 1 und/oder 2 sowie deren physiologisch verträgliche Salze, **dadurch gekennzeichnet, daß**
n für eine ganze Zahl 7, 8, 9 oder 10 steht,
m 1 bedeutet,
R¹ für (C₁-C₅)-Alkyl, Phenyl, Naphthyl oder einen Rest aus der Gruppe Piperidinyl, Pyrrolidinyl, Piperazinyl, Pyridinyl, Morpholinyl, Imidazolyl und Pyrazolyl steht,
R², R³, R⁴ und R⁵ xgleichzeitig für Wasserstoff, Natrium oder Lithium stehen.

4. Verbindungen der Formel I 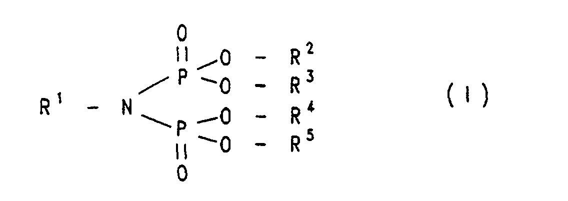 aus der Gruppe der Verbindungen
N-Cycloheptyl-imidobisphosphorsäuretetraethylester,
N-[2-(N',N'-Diisopropylamino)-ethyl]-imidobisphosphorsäuretetraethylester,
N-[2-(N'-Morpholino)-ethyl]-imidobisphosphorsäuretetraethylester,
N-[2-(2-Pyridyl)-ethyl]-imidobisphosphorsäuretetraethylester,
N-[3-(N'-Morpholino)-propyl-imidobisphosphorsäuretetraethylester,
N-[1-(Cyclohexyl)-ethyl]-imidobisphosphorsäuretetraethylester,
N-Heptyl-imidobisphosphorsäuretetraethylester,
N-Benzyl-imidobisphosphorsäuretetraethylester,
N-Benzyl-imidobisphosphorsäure,
N-[2-(N',N'-Diisopropylamino)-ethyl]-imidobisphosphorsäure,
N-[2-(N'-Morpholino)-ethyl]-imidobisphosphorsäure,
N-[2-(2-Pyridyl)-ethyl]-imidobisphosphorsäure,
N-[3-(N'-Morpholino)-propyl]-imidobisphosphorsäure,
N-Cycloheptyl-imidobisphosphorsäure-tetralithium,
N-Cyclohexyl-imidobisphosphorsäure-tetralithium,
N-[1-(Cyclohexyl)-ethyl]-imidobisphosphorsäure-tetralithium,
N-Heptyl-imidobisphosphorsäure-tetralithium,
N-Benzyl-imidobisphosphorsäure-tetralithium,
N-[2-(N',N'-Diisopropylamino)-ethyl]-imidobisphosphorsäure-tetratithium, und
N-[2-(N',N'-Dibutylamino)-ethyl]-imidobisphosphorsäure-tetralithium,
sowie deren physiologisch verträgliche Salze.

5. Verfahren zur Herstellung von Verbindungen der Formel I gemäß einem oder mehreren der Ansprüche 1 bis 4, **dadurch gekennzeichnet, daß** man
a) Imidobisphosphorigsäurealkylester der Formel III, 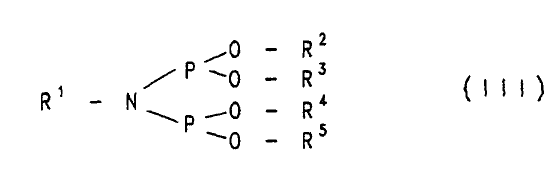 worin
R¹ die in den Ansprüchen 1 bis 4 angegebenen Bedeutungen haben und
R², R³, R⁴ und R⁵ für (C₁-C₅)-Alkyl stehen,
mit einem sauerstoffübertragenden Agenz zu Verbindungen der Formel I umsetzt,
b) gegebenenfalls die so erhaltenen Verbindungen in einem inerten Lösungsmittel mit Hilfe eines Alkyl-halo-silans zu Imidobisphosphorsäuretetratrimethylsilylestern umsetzt, und
c) anschließend gegebenenfalls hydrolysiert oder mit Hilfe eines basischen Salzes in die entsprechenden Salze überführt.

6. Arzneimittel, enthaltend eine wirksame Menge mindestens einer Verbindung der Formel I gemäß einem oder mehreren der Ansprüche 1 bis 4 oder mindestens eines physiologisch verträglichen Salzes einer solchen Verbindung, neben pharmazeutisch geeigneten und physiologisch verträglichen Hilfsstoffen und/oder Trägerstoffen und/oder Verdünnungsmitteln und/oder anderen Wirkstoffen.

7. Verfahren zur Herstellung eines Arzneimittel gemäß Anspruch 6, **dadurch gekennzeichnet, daß** man mindestens eine Verbindung der Formel I gemäß einem oder mehreren der Ansprüche 1 bis 4 und/oder mindestes ein physiologisch verträgliches Salz einer solchen Verbindung gegebenenfalls mit weiteren Hilfsstoffen und/oder Trägerstoffen in eine geeignete Darreichungsform bringt.

8. Verwendung der Verbindungen der Formel I gemäß einem oder mehreren der Ansprüche 1 bis 4 und/oder deren physiologisch verträglichen Salzen zur Herstellung eines Medikaments zur Prophylaxe oder Behandlung von Osteoporose oder Hypercalcemia.

9. Arzneimittel, enthaltend eine wirksame Menge mindestens einer Verbindung der Formel I, 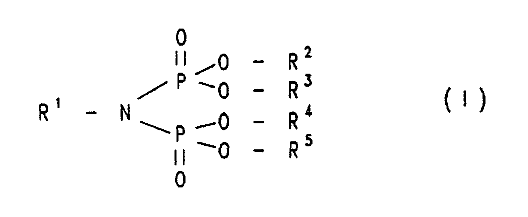 worin bedeuten:
R¹ = Methyl und R² - R⁵ gleichzeitig = Methyl, Ethyl, Propyl, Isopropyl, Butyl oder Isobutyl oder R² und R³ = Ethyl oder Isopropyl und R⁴ und R⁵ = Methyl;
R¹ = Ethyl und R² - R⁵ gleichzeitig = Ethyl, Propyl, Isopropyl, Butyl oder Isobutyl oder R² und R³ = Ethyl und R⁴ und R⁵ = Methyl;
R¹ = Isopropyl und R² und R³ = Isopropyl und R⁴ und R⁵ = Methyl;
R¹ = Butyl und R² - R⁵ gleichzeitig = Ethyl, Isopropyl oder Butyl;
R¹ = Isobutyl und R² - R⁵ gleichzeitig = Ethyl;
R¹ = Pentyl und R² - R⁵ gleichzeitig = Ethyl, Propyl oder Butyl;
R¹ = 1-tert. Butyl-1,1'-dimethyl-propyl und R² - R⁵ gleichzeitig = Ethyl;
R¹ = Phenyl und R² - R⁵ gleichzeitig = Propyl; oder
R¹ = Cyclohexyl und R² - R⁵ gleichzeitig = Ethyl,
neben pharmazeutisch geeigneten und physiologisch verträglichen Hilfsstoffen und/oder Trägerstoffen und/oder Verdünnungsmitteln und/oder anderen Wirkstoffen.

10. Verwendung der Verbindungen der Formel I gemäß Anspruch 9 zur Herstellung eines Medikaments zur Prophylaxe oder Behandlung von Osteoporose oder Hypercalcemia.

## Claims

1. A compound of the formula I 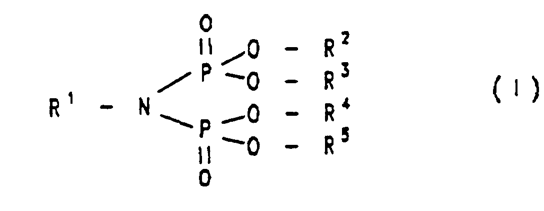 or its physiologically tolerable salts, where
R¹ is (C₁-C₁₀)-alkyl, (C₁-C₈)-alkanoyl, (C₆-C₁₀)-aryl or Het, each of which can be substituted one to six times by halogen, -OH, -NH₂, -NH-CO-R⁷, -O-CO-R⁷ and -N(R⁸)₂,
where R⁷ is (C₁-C₆)-alkyl and R⁸, independently of one another, is hydrogen or (C₁-C₃)-alkyl, and
Het is a monocyclic 5- to 6-membered or bicyclic 8- to 10-membered saturated, unsaturated or partially unsaturated heterocyclic radical having 1 to 3 identical or different heteroatoms selected from the group consisting of nitrogen, oxygen and sulphur,
or the radical of the formula II 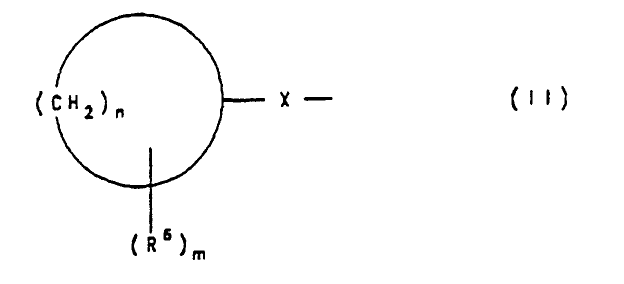 in which
n is an integer from 3 to 10,
m is zero to 3,
R⁶, independently of one another, is (C₁-C₆)-alkyl, which can be substituted one to six times by halogen or -NH-CO-R⁷ where R⁷ = (C₁-C₆)-alkyl and
X is absent or is (C₁-C₁₀)-alkyl, and
R², R³, R⁴ or R⁵, independently of one another, are hydrogen, (C₁-C₅)-alkyl, lithium, sodium, potassium or Si(R)₃, where R = (C₁-C₅)-alkyl,
compounds of the formula I being excluded where
R¹ = methyl and R²-R⁵ simultaneously = methyl, ethyl, propyl, isopropyl, butyl or isobutyl or R² and R³ = ethyl or isopropyl and R⁴ and R⁵ = methyl;
R¹ = ethyl and R²-R⁵ simultaneously = ethyl, propyl, isopropyl, butyl or isobutyl or R² and R³ = ethyl and R⁴ and R⁵ = methyl;
R¹ = isopropyl and R² and R³ = isopropyl and R⁴ and R⁵ = methyl;
R¹ = butyl and R²-R⁵ simultaneously = ethyl, isopropyl or butyl;
R¹ = isobutyl and R²-R⁵ simultaneously = ethyl;
R¹ = pentyl and R²-R⁵ simultaneously = ethyl, propyl or butyl;
R¹ = 1-tert-butyl-1,1'-dimethylpropyl and R²-R⁵ simultaneously = ethyl;
R¹ = dodecyl and R²-R⁵ simultaneously = butyl;
R¹ = phenyl and R²-R⁵ simultaneously = propyl; and
R¹ = cyclohexyl and R²-R⁵ simultaneously = ethyl.

2. A compound of the formula I as claimed in claim 1, or its physiologically tolerable salts, wherein
R¹ is (C₁-C₁₀)-alkyl, (C₁-C₈)-alkanoyl, (C₆-C₁₀)-aryl or Het, each of which can be substituted one to six times by
halogen, -OH, -NH₂, -NH-CO-R⁷, -O-CO-R⁷ and -N(R⁸)₂,
where R⁷ is (C₁-C₆)-alkyl and R⁸, independently of one another, is hydrogen or
(C₁-C₃)-alkyl, and
Het contains one or two nitrogen atoms,
or the radical of the formula II 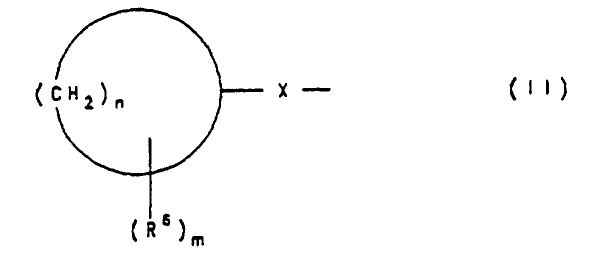 in which
n is an integer from 3 to 10,
m is 1,
R⁶ is (C₁-C₆)-alkyl which can be substituted one to six times by halogen or -NH-CO-R⁷ where R⁷ = (C₁-C₆)-alkyl, and
X is absent or (C₁-C₁₀)-alkyl, and
R² and R³, R⁴ and R⁵ are hydrogen, lithium, sodium or (C₁-C₅)-alkyl,
compounds of the formula I being excluded where
R¹ = methyl and R²-R⁵ simultaneously = methyl, ethyl, propyl, isopropyl, butyl or isobutyl or R² and R³ = ethyl or isopropyl and R⁴ and R⁵ = methyl;
R¹ = ethyl and R²-R⁵ simultaneously = ethyl, propyl, isopropyl, butyl or isobutyl or R² and R³ = ethyl and R⁴ and R⁵ = methyl;
R¹ = isopropyl and R² and R³ = isopropyl and R⁴ and R⁵ = methyl;
R¹ = butyl and R²-R⁵ simultaneously = ethyl, isopropyl or butyl;
R¹ = isobutyl and R²-R⁵ simultaneously = ethyl;
R¹ = pentyl and R²-R⁵ simultaneously = ethyl, propyl or butyl;
R¹ = 1-tert-butyl-1,1'-dimethylpropyl and R²-R⁵ simultaneously = ethyl;
R¹ = phenyl and R²-R⁵ simultaneously = propyl; and
R¹ = cyclohexyl and R²-R⁵ simultaneously = ethyl.

3. A compound of the formula I as claimed in claim 1 and/or 2, or its physiologically tolerable salts, wherein
n is an integer 7, 8, 9 or 10,
m is 1,
R¹ is (C₁-C₅)-alkyl, phenyl, naphthyl or a radical from the group consisting of piperidinyl, pyrrolidinyl, piperazinyl, pyridinyl, morpholinyl, imidazolyl and pyrazolyl,
R², R³, R⁴ and R⁵ simultaneously are hydrogen, sodium or lithium.

4. A compound of the formula I 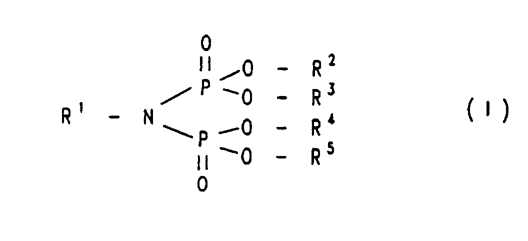 from the group consisting of the compounds
tetraethyl N-cycloheptylimidobisphosphate,
tetraethyl N-[2-(N',N'-diisopropylamino)ethyl]imidobisphosphate,
tetraethyl N-[2-(N'-morpholino)ethyl]imidobisphosphate,
tetraethyl N-[2-(2-pyridyl)ethyl]imidobisphosphate,
tetraethyl N-[3-(N'-morpholino)propyl]imidobisphosphate,
tetraethyl N-[1-(cyclohexyl)ethyl]imidobisphosphate,
tetraethyl N-heptylimidobisphosphate,
tetraethyl N-benzylimidobisphosphate,
N-benzylimidobisphosphoric acid,
N-[2-(N',N'-diisopropylamino)ethyl]imidobisphosphoric acid,
N-[2-(N'-morpholino)ethyl]imidobisphosphoric acid,
N-[2-(2-pyridyl)ethyl]imidobisphosphoric acid,
N-[3-(N'-morpholino)propyl]imidobisphosphoric acid, tetralithium N-cycloheptylimidobisphosphate,
tetralithium N-cyclohexylimidobisphosphate,
tetralithium N-[1-(cyclohexyl)ethyl]imidobisphosphate,
tetralithium N-heptylimidobisphosphate,
tetralithium N-benzylimidobisphosphate,
tetralithium N-[2-(N',N'-diisopropylamino)ethyl]imidobisphosphate, and
tetralithium N-[2-(N',N'-dibutylamino)ethyl]imidobisphosphate,
or its physiologically tolerable salts.

5. A process for the preparation of compounds of the formula I, as claimed in one or more of claims 1 to 4, which comprises
a) reacting alkyl imidobisphosphites of the formula III 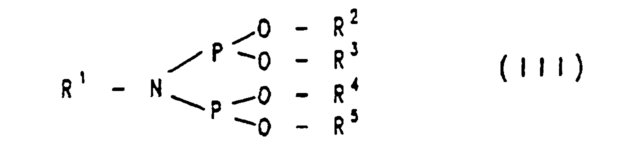 in which
R¹ has the meaning given in claims 1 to 4 and
R², R³, R⁴ and R⁵ are (C₁-C₅)-alkyl,
with an oxygen-transferring agent to give compounds of the formula I,
b) optionally reacting the compounds thus obtained in an inert solvent with the aid of an alkylhalosilane to give tetratrimethylsilyl imidobisphosphates, and
c) then optionally hydrolyzing or converting into the corresponding salts with the aid of a basic salt.

6. A pharmaceutical, containing an effective amount of at least one compound of the formula I as claimed in one or more of claims 1 to 4, or at least one physiologically tolerable salt of such a compound, in addition to pharmaceutically suitable and physiologically tolerable auxiliaries and/or excipients and/or diluents and/or other active compounds.

7. A process for the production of a pharmaceutical as claimed in claim 6, which comprises bringing at least one compound of the formula I as claimed in one or more of claims 1 to 4 and/or at least one physiologically tolerable salt of such a compound into a suitable administration form, if appropriate using further auxiliaries and/or excipients.

8. The use of a compound of the formula I as claimed in one or more of claims 1 to 4 and/or its physiologically tolerable salts for the production of a medicament for the prophylaxis or treatment of osteoporosis and hypercalcemia.

9. A pharmaceutical, containing an effective amount of at least one compound of the formula I 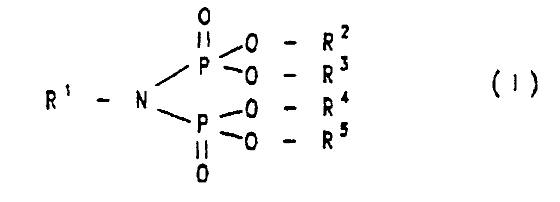 in which:
R¹ = methyl and R²-R⁵ simultaneously = methyl, ethyl, propyl, isopropyl, butyl or isobutyl or R² and R³ = ethyl or isopropyl and R⁴ and R⁵ = methyl;
R¹ = ethyl and R²-R⁵ simultaneously = ethyl, propyl, isopropyl, butyl or isobutyl or R² and R³ = ethyl and R⁴ and R⁵ = methyl;
R¹ = isopropyl and R² and R³ = isopropyl and R⁴ and R⁵ = methyl;
R¹ = butyl and R²-R⁵ simultaneously = ethyl, isopropyl or butyl;
R¹ = isobutyl and R²-R⁵ simultaneously = ethyl;
R¹ = pentyl and R²-R⁵ simultaneously = ethyl, propyl or butyl;
R¹ = 1-tert-butyl-1,1'-dimethylpropyl and R²-R⁵ simultaneously = ethyl;
R¹ = phenyl and R²-R⁵ simultaneously = propyl; or
R¹ = cyclohexyl and R²-R⁵ simultaneously = ethyl,
in addition to pharmaceutically suitable and physiologically tolerable auxiliaries and/or excipients and/or diluents and/or other active compounds.

10. The use of a compound of the formula I as claimed in claim 9 for the production of a medicament for the prophylaxis or treatment of osteoporosis or hypercalcemia.

## Revendications

1. Composés de formule I 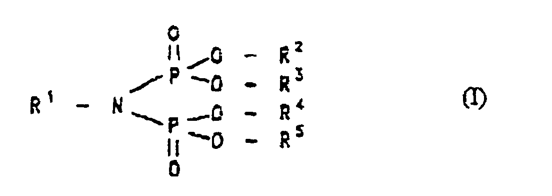 et leurs sels physiologiquement acceptables, où
R¹ représente un reste alkyle en C₁-C₁₀, alcanoyle en C₁-C₈, aryle en C₆-C₁₀ ou Het, qui peut être substitué 1 à 6 fois par des restes halogéno, -OH, -NH₂, -NH-CO-R⁷. -O-CO-R⁷ et-N(R⁸)₂,
où R⁷ représente un reste alkyle en C₁-C₆ et les R⁸ sont indépendamment l'un de l'autre atome d'hydrogène ou un reste alkyle en C₁-C₃, et
Het représente un reste hétérocyclique monocyclique de 5 à 6 chaînons ou bicyclique de 8 à 10 chaînons, saturé, insaturé ou partiellement insaturé, contenant 1 à 3 hétéroatomes identiques ou différents choisis dans la série de l'azote de l'oxygène et du soufre,
ou représente le reste de formule II 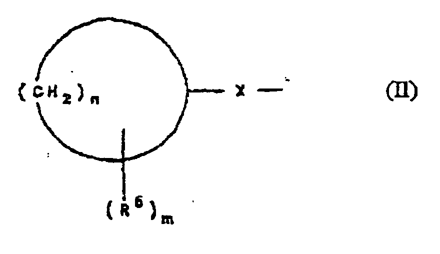
dans laquelle
n représente un nombre entier de 3 à 10.
m est un nombre de 0 à 3,
les R⁶ eprésentent, indépendamment les uns des autres, un reste alkyle en C₁-C₆ éventuellement substitué 1 à 6 fois par des restes halogéno ou -NH-CO-R⁷, R⁷ représentant un reste alkyle en C₁-C₆, et
X es absent ou représente un reste alkyle en C₁-C₁₀, et
R², R³, R⁴ et R⁵ représentent, indépendamment les uns des autres, un atome d'hydrogène, un reste akyle en C₁-C₅, un arome de lithium, de sodium ou de potassium ou un reste Si(R)₃ dans lequel R représente un reste alkyle en C₁-C₅,
à l'exception des composés de formule I dans lesquels
R¹ est un este méthyle et R² à R⁵ sont en même temps des restes méthyle, éthyle, propyle, isopropyle, butyle ou isobutyle ou R² et R³ sont des restes éthyle ou isopropyle et R⁴ et R⁵ sont des restes méthyle,
R¹ est un reste éthyle et R² à R⁵ sont en même temps des restes éthyle, propyle, isopropyle, butyle ou isobutyle ou R² et R³ sont des restes éthyle et R⁴ et R⁵ sont des restes méthyle,
R¹ est un isopropyle et R² et R³ sont des restes isopropyle et R⁴ et R⁵ sont des restes méthyle,
R¹ est un reste butyle et R² à R⁵ sont en même temps des restes éthyle, isopropyle ou butyle,
R¹ est un reste isobutyle et R² à R⁵ sont en même temps des restes éthyle,
R¹ est un reste pentyle et R² à R⁵ sont en même temps des restes éthyle, propyle ou butyle
R¹ est un reste 1-tert-butyl-1,1'-diméthylpropyle et R² à à R⁵ sont en même temps des restes éthyle,
R¹ est un reste dodécyle et R² à R⁵ sont en même temps des restes butyle,
R¹ est un este phényle et R² à R⁵ sont en même temps des restes propyle, et
R¹ est un reste cyclohexyle et R² à R⁵ sont en même temps des restes éthyle.

2. Composés de formule I selon la revendication 1 et leurs sels physiologiquement acceptables, **caractérisés en ce que**
R¹ représente un reste alkyle en C₁-C₁₀, alcanoyle en C₁-C₈, aryle en C₆-C₁₀ ou Het, qui peut être substitué 1 à 6 fois par des restes halogéno, -OH, -NH₂, -NH-CO-R⁷, -O-CO-R⁷ et -N(R³)₂,
où R⁷ représente un reste alkyle en C₁-C₆ et les R⁸ sont indépendamment l'un de l'autre un atome d'hydrogène ou un reste alkyle en C₁-C₃, et
Het contient un ou deux atomes d'azote,
ou représente le reste de formule II 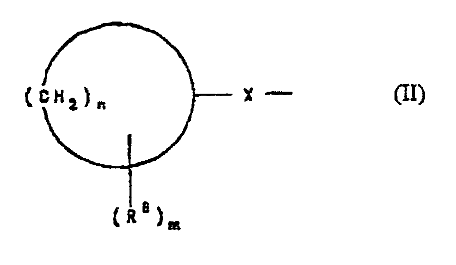 dans laquelle
n représente un nombre entier de 3 à 10,
m est égal à 1,
les R⁶ représentent des restes alkyle en C₁-C₆ éventuellement substitués 1 à 6 fois par des restes halogéno ou -NH-CO-R⁷, R⁷ représentant un reste alkyle en C₁-C₆, et
X est absent ou représente un reste akyle en C₁-C₁₀, et
R² et R³, R⁴ et R⁵ représentent des atomes d'hydrogène, de lithium ou de sodium ou des restes alkyle en C₁-C₅,
à l'exception des composés de formule I dans lesquels
R¹ est un este méthyle et R² à R⁵ sont en même temps des restes méthyle, éthyle, propyle, isopropyle, butyle ou isobutyle ou R² et R³ sont des restes éthyle ou isopropyle et R⁴ et R⁵ sont des restes méthyle,
R¹ est un reste éthyle et R² à R⁵ sont en même temps des restes éthyle, propyle, isopropyle, butyle ou isoburyle ou R² et R³ sont des restes éthyle et R⁴ et R⁵ sont des restes méthyle,
R¹ est un reste isopropyle et R² et R³ sont des restes isopropyle et R⁴ et R⁵ sont des restes méthyle,
R¹ est un reste butyle et R² à R⁵ sont en même temps des restes éthyle, isopropyle ou butyle,
R¹ est un reste isobutyle et R² à R⁵ sont en même temps des restes éthyle,
R¹ est un reste pentyle et R² à R⁵ sont en même temps des restes éthyle, propyle ou butyle,
R¹ est un reste 1-tert-butyl-1,1'-diméthylpropyle et R² à R⁵ sont en même temps des restes éthyle,
R¹ est un este phényle et R² à R⁵ sont en même temps des restes propyle, et
R¹ est un reste cyclohexyle et R² à R⁵ sont en même temps des restes éthyle.

3. Composés de formule I selon la revendication 1 et/ou 2 et leurs sels physiologiquement acceptables, **caractérisés en ce que**
n est un nombre entier égal à 7, 8, 9 ou 10,
m est égal à 1,
R¹ représente un reste alkyle en C₁-C₅, phényle, naphtyle, ou un reste du groupe constitué par les restes pipéridinyle, pyrrolidinyle, pipérazinyle, pyridinyle, morpholinyle, imidazolyle et pyrazolyle,
R², R³, R⁴ et R⁵ représentent en même temps des atomes d'hydrogène, de sodium ou de lithium.

4. Composés de formule I 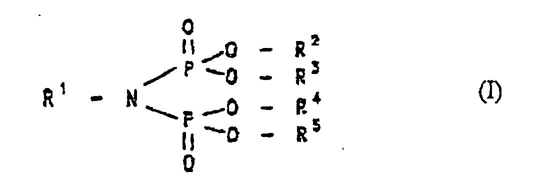 appartenant au groupe de composés suivants:
le N-cycloheptylimidobisphosphate de tétraélhyle,
le N-[2-(N',N'-diïsopropylamino)éthyl]imidobisphosphate de tétraéthyle,
le N-[2+(N'-morpholino)éthyl]imidobisphosphate de tétraéthyle,
le N-[2-(2-pyridyl)éthyl]imidobisphosphate de tétraéthyle,
le N-[3-(N'-morpholino)propylimidobisphosphare de tétraéthyle,
le N-[1-(cyclohexyl)éthyl]imidobisphosphare de tétraéthyle,
le N-heptylimidobisphosphate de tétraéthyle,
le N-benzylimidobisphosphate de tétraéthyle,
l'acide N-benzylimidobisphosphorique,
l'acide N-[2-(N',N'-diisopropylamino)éthyl]imidobisphosphorique.
l'acide N-[2-(N'-morpholino)éthyl]imidobisphosphorique,
l'acide N-[2-(2-pyridyl)éthyl]imidobisphosphorique,
l'acide N-[3-(N'-morpholino)propyl]imidobisphosphorique,
le N-cycloheptylimidobisphosphate de tétralithium,
le N-cyclohexylimidobisphosphate de tétralithium,
le N-[1-(cyclohexyl)éthyl]imidobisphosphate de tétralithium,
le N-heptylimidobisphosphate de tétralithium,
le N-benzylimidobisophosphate de tétralithium,
le N-[2-(N',N'-diisopropylammo)éthyl]imidobisphosphate de tétralithium,
le N-[2-(N','-dibutylamino)éthyl]imidobisphosphate de tétralithium.
et leurs sels physiologiquement acceptables.

5. Procédé de préparation de composés de formule I selon l'une ou plusieurs des revendications 1 à 4, **caractérisé en ce que**
a) on fait réagir un imidobisphosphite d'alkyle de formule III 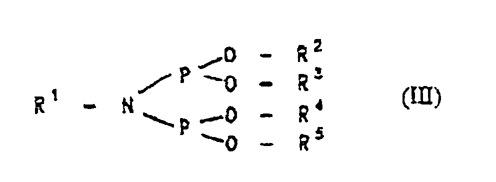 dans laquelle
R¹ a la signification indiquée dans les revendications 1 à 4 et
R², R³, R⁴ et R⁵ représentent des restes alkyle en C₁-C₅,
avec un agent de transfert d'oxygène pour obtenir un composé de formule I,
b) on transforme éventuellement le composé ainsi obtenu en un imidobisphosphate de tétra(triméthylsilyle) dans un solvant inerte à l'aide d'un alkylhalogénosilane, et
c) on procède éventuellement ensuite à une hydrolyse ou on forme le sel correspondant à l'aide d'un sel basique.

6. Médicaments contenant une quantité active d'au moins un composé de formule I selon l'une ou plusieurs des revendications 1 à 4 ou d'au moins un sel physioloquement acceptable d'un tel composé, avec des additifs et/ou des supports et/ou des diluants et/ou d'autres ingrédients actifs pharmaceutiquement appropriés et physiologiquement acceptables.

7. Procédé de préparation d'un médicament selon la revendication 6, **caractérisé en ce que** l'on met au moins un composé de formule I selon l'une ou plusieurs des revendications 1 à 4 et/ou au moins un sel physiologiquement acceptable d'un tel composé, éventuellement avec d'autres additifs et/ou supports, sous une forme d'administration appropriée.

8. Utilisation des composés de formule I selon l'une ou plusieurs des revendications 1 à 4 et/ou de leurs sels physiologiquement acceptables pour la préparation d'un médicament destiné à la prophylaxie ou au traitement de l'ostéoporose ou de l'hypercalcémie.

9. Médicaments contenant une quantité active d'au moins un composé de formule I 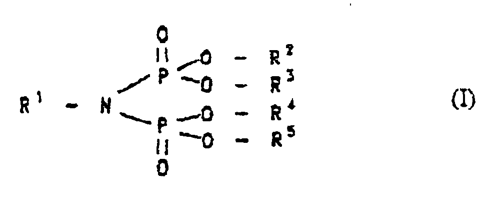 dans laquelle
R¹ est un reste méthyle et R² à R⁵ sont en même temps des restes méthyle, éthyle, propyle, isopropyle, butyle ou isobutyle ou R² et R³ sont des restes éthyle ou isopropyle et R⁴ et R⁵ sont des restes méthyle,
R¹ est un reste éthyle et R² à R⁵ sont en même temps des restes éthyle, propyle, isopropyle, butyle ou isobutyle ou R² et R³ sont des restes éthyle et R⁴ et R⁵ sont des restes méthyle,
R¹ est un reste isopropyle er R² et R³ sont des restes isopropyle et R⁴ et R⁵ sont des restes méthyle,
R¹ est un reste butyle et R² à R⁵ sont en mème temps des restes éthyle, isopropyle ou butyle,
R¹ est un r ste isobutyle et R² à R⁵ sont en même temps des restes éthyle,
R¹ est un reste pentyle et R² à R⁵ sont en même temps des restes éthyle, propyle ou butyle,
R¹ est un reste 1-tert-butyl-1,1'-diméthylpropyle et R² à R⁵ sont en même temps des restes éthyle,
R¹ est un reste phényle et R² à R⁵ sont en même temps des restes propyle, et
R¹ est un reste cyclohexyle et R² à R⁵ sont en même temps des restes éthyle,
avec des additifs et/ou des supports et/ou des diluants et/ou d'autres ingrédients actifs pharmaceutiquement appropriés et physiologiquement acceptables.

10. Utilisation des composés de formule I selon la revendication 9 pour la préparation d'un médicament destiné à la prophylaxie ou au traitement de l'ostéoporose ou de l'hypercalcémie.
